# EUROPEAN PATENT APPLICATION

(11) **EP 4 813 327 A1**
(43) Date of publication of application: **30.09.2026**
(21) Application number: 26159399.0
(22) Date of filing: 18.02.2026
(51) Int. Cl.: A61B 17/70

(54) **SPINAL FIXATION ROD**

(30) Priority: 27.03.2025 JP 2025053791
(71) Applicant: Globeride, Inc., Higashikurume-shi, Tokyo 203-8511 (JP)
(72) Inventor: OIKAWA, Katsuhiro, Higashikurume-shi, 203-8511 (JP); KUSAKA, Hiroshi, Higashikurume-shi, 203-8511 (JP); KUSUMOTO, Harunobu, Higashikurume-shi, 203-8511 (JP)
(74) Representative: Boult Wade Tennant LLP

(57) **Abstract**

A fixation rod according to an embodiment of the present invention is a fixation rod clamped by a fastening member fastened to a spine, in which a plurality of fiber layers are formed such that the plurality of fiber layers are laminated to overlap in a radial direction of the fixation rod as viewed in a cross section perpendicular to an axial direction of the fixation rod, a marking portion indicating a clamping location by the fastening member is formed in the fixation rod, and the marking portion is configured to indicate a clamping location in which a clamping direction by the fastening member at the clamping location is a direction intersecting an interlayer direction of the plurality of fiber layers.

## Description

### BACKGROUND OF THE INVENTION

### 1. TECHNICAL FIELD

The present invention relates to a fixation rod used in a fixation device for fixing the spine.

### 2. DESCRIPTION OF THE RELATED ART

In the related art, various fixation rods have been known as fixation devices for fixing the spine.

For example, as such a fixation rod, JP 2024-546988 A discloses that, in manufacturing an implant, a plurality of prepregs, particularly unidirectional prepregs, are used, these prepregs are pressure-grouted with one another under pressure and heat, the prepregs are fiber-matrix semi-finished products, that is, semi-finished products including reinforcing fibers disposed in a plastic matrix, the reinforcing fibers are carbon fibers, the prepregs may be present, for example, in the form of strands, bands, or plates, the prepregs are continuously fused together to form a final shape of the implant by pressure-grouting the prepregs under pressure and temperature, and after the pressure-grouting, the prepregs form an implant body.

### RELEVANT REFERENCES

### LIST OF RELEVANT PATENT LITERATURE

Patent Literature 1: Japanese Patent Application Publication No. 2024-546988

### SUMMARY OF THE INVENTION

However, the rod disclosed in JP 2024-546988 A still has room for improvement from the viewpoint of achieving more stable and reliable use when the rod is fixed to a screw head.

An object of the present invention is to provide a fixation rod that enables more stable and reliable use during screw fixation. Other objects of the present invention will become apparent by referring to the entirety of this specification.

A fixation rod according to an embodiment of the present invention is a fixation rod clamped by a fastening member fastened to a spine, in which a plurality of fiber layers are formed such that the plurality of fiber layers are laminated to overlap in a radial direction of the fixation rod as viewed in a cross section perpendicular to an axial direction of the fixation rod, a marking portion indicating a clamping location by the fastening member is formed in the fixation rod, and the marking portion is configured to indicate a clamping location in which a clamping direction by the fastening member at the clamping location is a direction intersecting an interlayer direction of the plurality of fiber layers.

In the fixation rod according to the embodiment of the present invention, the marking portion is formed as an indication portion capable of specifying the clamping location or a shape portion capable of specifying the clamping location.

In the fixation rod according to the embodiment of the present invention, the indication portion is a marker provided on an outer peripheral surface of the fixation rod.

In the fixation rod according to the embodiment of the present invention, the shape portion is a clamping surface or a curved shape provided on an outer peripheral surface of the fixation rod.

In the fixation rod according to the embodiment of the present invention, the curved shape includes a shape curved in an arcuate shape.

According to the embodiment of the present invention, it is possible to provide a fixation rod that enables more stable and reliable use during screw fixation.

### BRIEF DESCRIPTION OF THE DRAWINGS

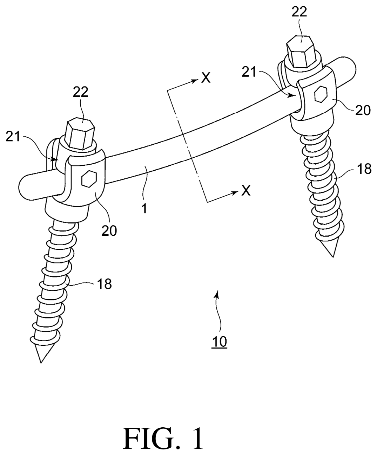

FIG. 1 is a diagram illustrating a spinal fixation device 10 comprising a fixation rod according to an embodiment of the present invention;

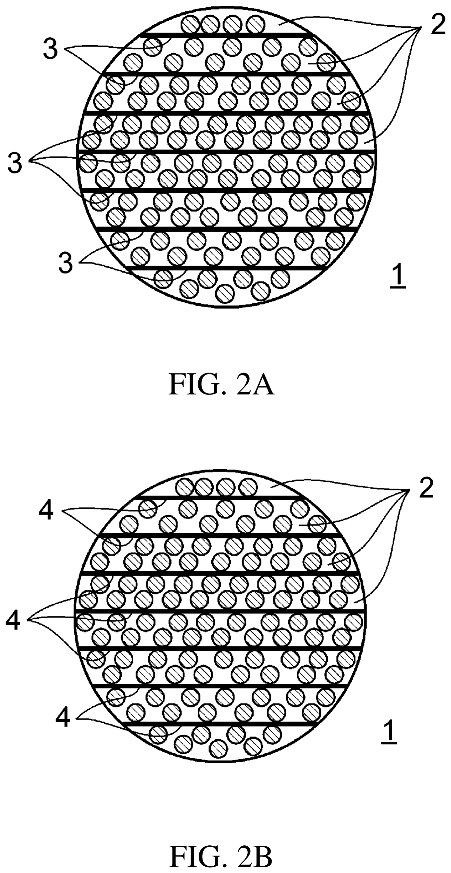

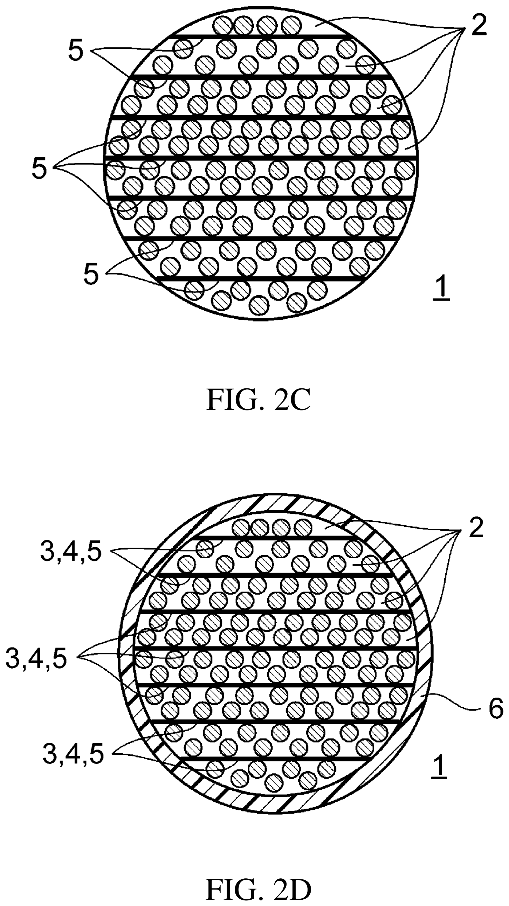

FIG. 2A is a diagram schematically illustrating a cross section of the fixation rod according to the embodiment of the present invention, taken on a plane perpendicular to the central axis thereof, FIG. 2B is a diagram schematically illustrating a cross section of the fixation rod according to the embodiment of the present invention, taken on a plane perpendicular to the central axis thereof, FIG. 2C is a diagram schematically illustrating a cross section of the fixation rod according to the embodiment of the present invention, taken on a plane perpendicular to the central axis thereof, and FIG. 2D is a diagram schematically illustrating a cross section of the fixation rod according to the embodiment of the present invention, taken on a plane perpendicular to the central axis thereof;

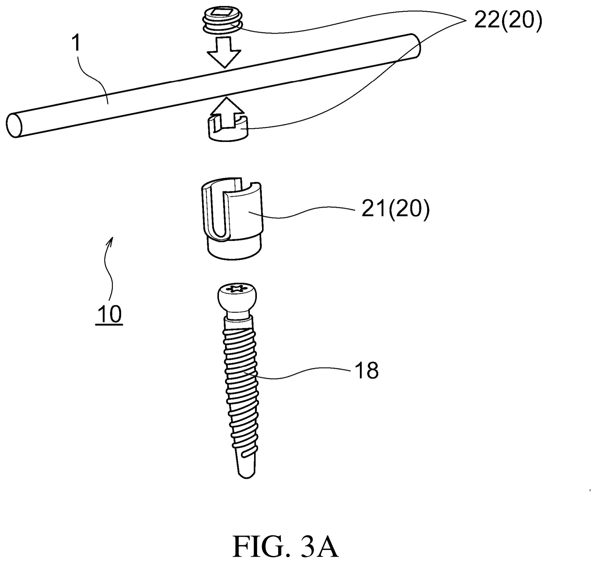

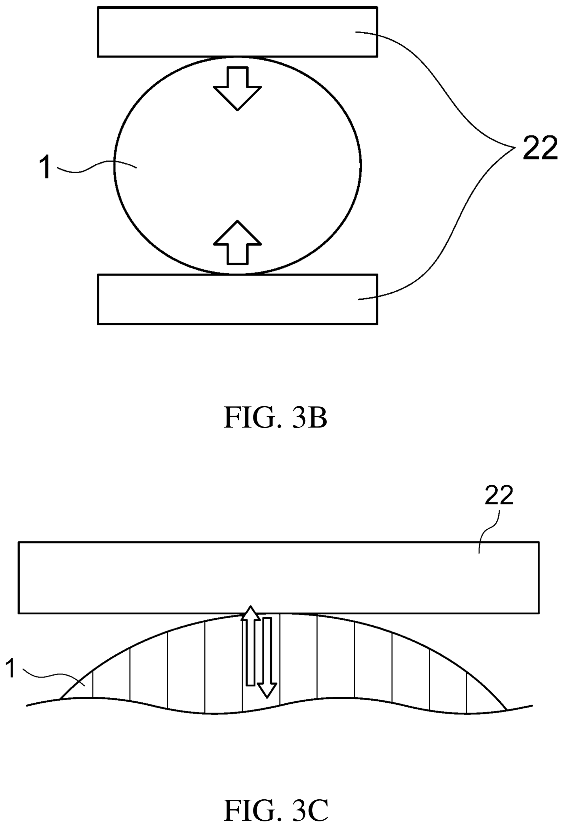

FIG. 3A is a diagram illustrating the spinal fixation device 10 comprising a fixation rod 1 according to the embodiment of the present invention, FIG. 3B is a schematic diagram (cross-sectional view) illustrating a direction of a shear force acting on the fixation rod 1 according to the embodiment of the present invention, and FIG. 3C is a schematic diagram (cross-sectional view) illustrating an interlayer direction (up-down direction in the illustrated example) of a fiber layer of the fixation rod 1 according to the embodiment of the present invention and a direction of a shear force;

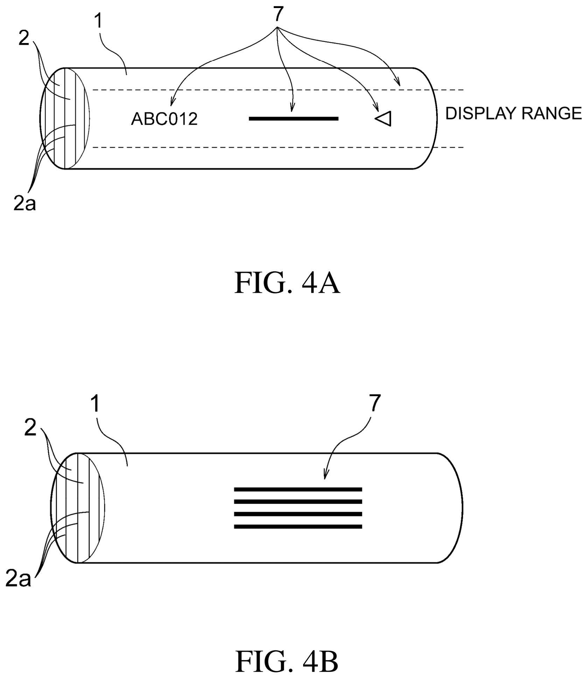

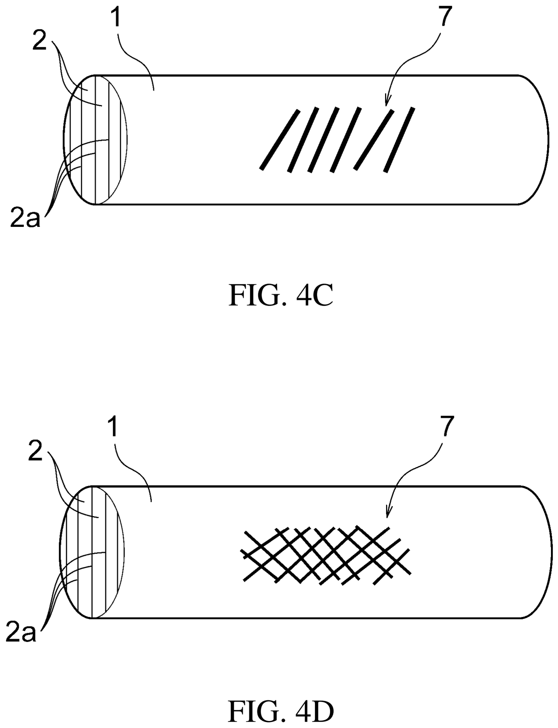

FIG. 4A is a diagram illustrating a marking portion in the fixation rod 1 according to the embodiment of the present invention, FIG. 4B is a diagram illustrating another form of the marking portion in the fixation rod 1 according to the embodiment of the present invention, FIG. 4C is a diagram illustrating another form of the marking portion in the fixation rod 1 according to the embodiment of the present invention, and FIG. 4D is a diagram illustrating another form of the marking portion in the fixation rod 1 according to the embodiment of the present invention;

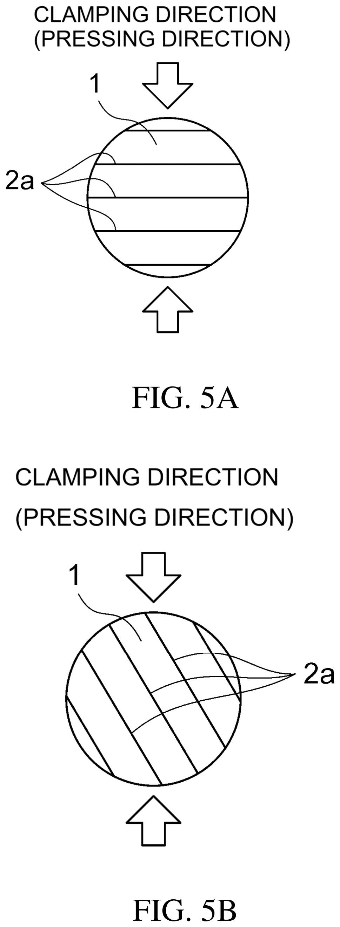

FIG. 5A is a diagram illustrating a relationship between the interlayer direction (direction of a boundary surface) of the fiber layer in the fixation rod 1 according to the embodiment of the present invention and a clamping direction (pressing direction) by a pressing member 22 (not illustrated), and FIG. 5B is a diagram illustrating a relationship between the interlayer direction (direction of a boundary surface) of the fiber layer in the fixation rod 1 according to the embodiment of the present invention and the clamping direction (pressing direction) by the pressing member 22 (not illustrated); and

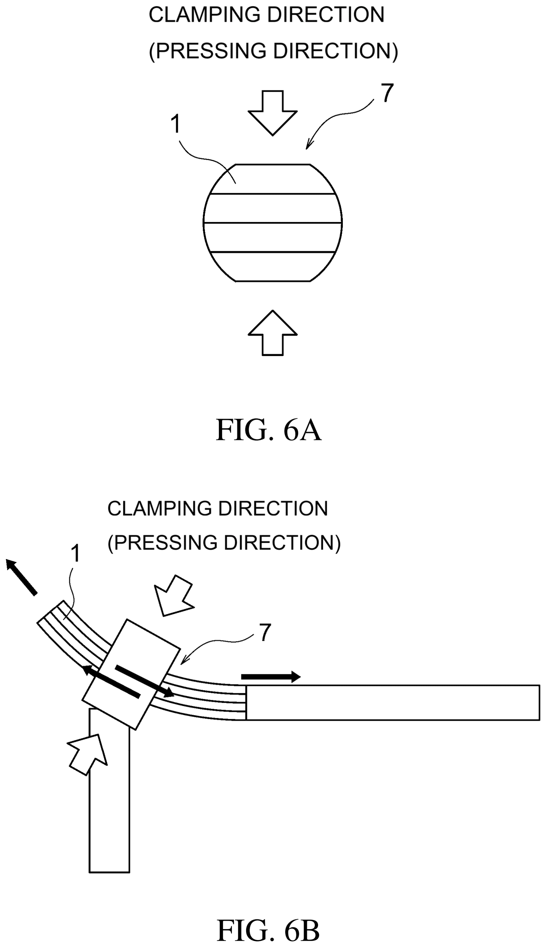

FIG. 6A is a diagram illustrating the marking portion in the fixation rod 1 according to the embodiment of the present invention, and FIG. 6B is a diagram illustrating the marking portion in the fixation rod 1 according to the embodiment of the present invention.

### DETAILED DESCRIPTION

Hereinafter, an embodiment of a fixation rod according to the present invention will be specifically described with reference to the accompanying drawings. Constituent elements common to a plurality of drawings are denoted by the same reference numerals throughout the plurality of drawings. It should be noted that the drawings are not always illustrated in a precise scale for the convenience of description.

FIG. 1 is a diagram illustrating a spinal fixation device 10 comprising a fixation rod 1 according to an embodiment of the present invention. As illustrated in the drawing, the spinal fixation device 10 comprises a plurality of screw members 18 (two screw members 18 in the illustrated example) to be fixed to bones of the spine; a plurality of rod fixing members 20 (two rod fixing members 20 in the illustrated example) which are attached to the screw members 18 and each of which comprises a recess portion 21 for receiving the fixation rod 1 and a pressing member 22, and the fixation rod 1 that is inserted into the recess portions 21 of the plurality of rod fixing members 20 and fixed by the pressing members 22.

Next, with reference to FIGS. 2A, 2B, 2C, and 2D, an example of the fixation rod 1 according to the embodiment of the present invention used in the spinal fixation device 10 will be described. FIGS. 2A, 2B, 2C, and 2D are cross-sectional views of the fixation rod 1 illustrated in FIG. 1, taken along the X-X cross section illustrated in FIG. 1. As illustrated in the drawing, the fixation rod 1 according to the embodiment of the present invention is formed, as viewed in a cross section, by alternately laminating a plurality of layers comprising a reinforcing fiber layer 2 and any one of a resin layer 3 (example illustrated in FIGS. 2A to 2D), a reinforcing fiber layer 4 (example illustrated in FIG. 3) made of fibers different from the fibers of the reinforcing fiber layer 2, and a reinforcing fiber layer 5 (example illustrated in FIGS. 4A to 4D) having fibers obliquely oriented with respect to a fiber direction of the reinforcing fiber layer 2. In this manner, it is possible to provide a fixation rod that reduces damage during screw fixation, exhibits high rigidity and high durability against deformation loads, and significantly improves safety even at the time of fracture. In particular, at the time of fracture of the fixation rod 1 according to the embodiment of the present invention, by concentrating stress on any of the resin layer sandwiched between the reinforcing fiber layers, the reinforcing fiber layer different from the fibers of the reinforcing fiber layer, or the reinforcing fiber layer having fibers obliquely oriented with respect to the fiber direction of the reinforcing fiber layer, it is possible to reduce a tendency for the reinforcing fiber layer to form spike-like projections when the rod fractures, and thus it is possible to significantly enhance safety for the human body.

As illustrated in FIG. 2D, as viewed in a cross section, the fixation rod 1 according to the embodiment of the present invention is formed by alternately laminating a plurality of layers comprising the reinforcing fiber layer 2 and any one of the resin layer 3, the reinforcing fiber layer 4 made of fibers different from the fibers of the reinforcing fiber layer 2, and the reinforcing fiber layer 5 having fibers obliquely oriented with respect to the fiber direction of the reinforcing fiber layer 2, and a coating layer 6 is further formed on an outer surface of the fixation rod 1. The coating layer 6 may be, for example, a resin identical to the resin of the layers used in the above-described fixation rod 1, but is not limited thereto. In this manner, when the rod fractures, a tendency for the reinforcing fiber layer to form spike-like projections can be further reduced, and thus it is possible to further enhance safety for the human body.

Here, the reinforcing fiber layer 2 and any one of the resin layer 3 (example illustrated in FIGS. 2A to 2D), the reinforcing fiber layer 4 (example illustrated in FIG. 3) made of fibers different from the fibers of the reinforcing fiber layer 2, and the reinforcing fiber layer 5 (example illustrated in FIGS. 4A to 4D) having fibers obliquely oriented with respect to the fiber direction of the reinforcing fiber layer 2 are formed to have a thickness of 0.01 mm to 0.25 mm; however, the thickness of the layer may vary depending on a location of the layer (that is, the layer may be formed to have some thin portions and some thick portions). The layer may also be formed discontinuously (that is, not only may the layer have some thin portions and some thick portions, but portions in which the thickness of the layer is zero may also be interposed). In addition, the fixation rod 1 according to the embodiment of the present invention may be formed, as viewed in a cross section, by laminating a plurality of reinforcing fiber layers 2, and is not intended to be limited to a particular embodiment.

In the fixation rod 1 according to the embodiment of the present invention, the reinforcing fiber layer 2 is a fiber-reinforced resin, in which the fibers include carbon, glass, boron, SiC, or aramid, and the resin includes epoxy, phenolic resin, unsaturated polyester, PA, PC, PPSU, POM, PP, PE, ABS, PS, PAEK, or PEEK. In this manner, it is possible to increase bending rigidity and enhance strength of the fixation rod 1.

In the fixation rod 1 according to the embodiment of the present invention, the resin of the resin layer 3 includes epoxy, phenolic resin, unsaturated polyester, PA, PC, PPSU, POM, PP, PE, ABS, PS, PAEK, or PEEK.

In the fixation rod 1 according to the embodiment of the present invention, the reinforcing fiber layer 4 made of fibers different from the fibers of the reinforcing fiber layer 2 is a fiber-reinforced resin, in which the fibers include carbon, glass, boron, SiC, or aramid, and the resin includes epoxy, phenolic resin, unsaturated polyester, PA, PC, PPSU, POM, PP, PE, ABS, PS, PAEK, or PEEK. In this manner, it is possible to increase bending rigidity and enhance strength of the fixation rod. The reason for using a reinforcing fiber layer made of fibers different from the fibers of the reinforcing fiber layer is that, by using different materials, it is possible to impart different properties, such as flexibility and vibration damping characteristics, in addition to rigidity and strength.

Next, with reference to FIGS. 3A to 3C, a fixation state of the fixation rod 1 by the spinal fixation device 10 and a mechanism of generation of a shear force according to the embodiment of the present invention will be described. FIG. 3A is a diagram illustrating the spinal fixation device 10 comprising the fixation rod 1 according to the embodiment of the present invention, FIG. 3B is a schematic diagram (cross-sectional view) illustrating a direction of a shear force acting on the fixation rod 1 according to the embodiment of the present invention, and FIG. 3C is a schematic diagram (cross-sectional view) illustrating an interlayer direction (up-down direction in the illustrated example) of a fiber layer of the fixation rod 1 according to the embodiment of the present invention and a direction of a shear force. As illustrated in FIG. 3A, the spinal fixation device 10 comprises the screw member 18 (one screw member 18 in the illustrated example) fixed to a bone of the spine, the rod fixing member 20 (one rod fixing member 20 in the illustrated example) attached to the screw member 18 and comprising the recess portion 21 for receiving the fixation rod 1 and the pressing member 22 (in the illustrated example, the pressing member 22 includes two members), and the fixation rod 1 that is inserted into the recess portions 21 of the plurality of rod fixing members 20 and fixed by the pressing member 22. Here, a contact surface of the pressing member 22 (in a case where the pressing member 22 includes two members as in the illustrated example, at least one of the pressing members 22), which is in contact with the outer surface of the fixation rod 1, may be formed to conform to an outer surface shape (outer surface profile) of the fixation rod 1, and for example, the contact surface may be formed in an arcuate shape (in a case where the contact surface is viewed from a side).

As illustrated in FIG. 3B, regarding the fixation rod 1 according to the embodiment of the present invention, when the screw member 18 (not illustrated) of the spinal fixation device 10 is fastened, the fixation rod 1 is clamped by the pressing member 22, and the fixation rod 1 receives a pressing force from the pressing member 22 in the clamping direction thereof (in the examples of FIGS. 3A and 3B, the up-down direction (although not illustrated, an extending direction of the screw member 18)). Then, as illustrated in FIG. 3C, when the fixation rod receives the pressing force by the pressing member 22, a shear force is generated in the clamping direction (crushing direction) described above at a boundary region between a contact portion of the fixation rod 1 with the pressing member 22 (a contact portion of the fixation rod 1 contacted by the pressing member 22) and a non-contact portion thereof. When a direction of the shear force is aligned with an interlayer direction (the up-down direction in the illustrated example) 2a of fiber layers of the fixation rod 1 according to the embodiment of the present invention, the fixation rod 1 is more likely to undergo failure due to the shear force.

Next, the fixation rod 1 according to the embodiment of the present invention will be further described with reference to FIGS. 4A to 6B. FIG. 4A is a diagram illustrating a marking portion in the fixation rod 1 according to the embodiment of the present invention, FIG. 4B is a diagram illustrating another form of the marking portion in the fixation rod 1 according to the embodiment of the present invention, FIG. 4C is a diagram illustrating another form of the marking portion in the fixation rod 1 according to the embodiment of the present invention, and FIG. 4D is a diagram illustrating another form of the marking portion in the fixation rod 1 according to the embodiment of the present invention. FIGS. 5A and 5B are diagrams illustrating a relationship between the interlayer direction (direction of a boundary surface) of the fiber layer in the fixation rod 1 according to the embodiment of the present invention and the clamping direction (pressing direction) by the pressing member 22 (not illustrated). FIG. 6A is a diagram illustrating the marking portion in the fixation rod 1 according to the embodiment of the present invention, and FIG. 6B is a diagram illustrating the marking portion in the fixation rod 1 according to the embodiment of the present invention.

As illustrated in FIGS. 1 and 4A to 4D, the fixation rod 1 according to the embodiment of the present invention is the fixation rod 1 clamped by a fastening member (the rod fixing member 20 described above) fastened to the spine, a plurality of fiber layers (reinforcing fiber layers) 2 are formed such that the plurality of fiber layers are laminated to overlap in a radial direction of the fixation rod as viewed in a cross section (X-X cross section in FIG. 1) perpendicular to an axial direction (extending direction) of the fixation rod 1, a marking portion 7 (in the illustrated example, four forms of a character string, a line, a triangle, and dotted lines indicating a display range are illustrated simultaneously, and surface texturing or matte finishing may be applied as appropriate) indicating a clamping location (pressing location) by the fastening member (the rod fixing member 20 described above) is formed in the fixation rod 1, and the marking portion 7 is configured to indicate a clamping location in which the clamping direction by the fastening member (the rod fixing member 20 described above) at the clamp location is a direction intersecting an interlayer direction (direction of a boundary surface to be described later) 2a of the plurality of fiber layers 2. Here, as illustrated in FIGS. 5A and 5B, the "interlayer direction of the plurality of fiber layers" refers to a direction (extending direction) of the boundary surface between two adjacent fiber layers (of the interlayer) (in the example of FIG. 5A, a left-right direction on the paper, and in the example of FIG. 5B, a direction from the upper left to the lower right on the paper), and the "clamping direction" refers to a direction in which the fixation rod 1 is clamped (pressed) by the pressing member 22 (not illustrated) (in the illustrated examples, the up-down direction on the page). The term "direction intersecting" refers to a direction (for example, the directions illustrated in FIGS. 5A and 5B) in which the clamping direction by the fastening member (the rod fixing member 20 described above) at the clamping location is not the same as the interlayer direction (direction of the boundary surface) of the plurality of fiber layers 2.

According to the fixation rod 1 of the embodiment of the present invention, it is possible to provide a fixation rod that enables more stable and reliable use during screw fixation. More specifically, since a clamping (fixing) position of the fastening member (the rod fixing member 20 described above) with respect to the fixation rod 1 can be easily recognized, this facilitates an operation by an operator and also allows the fixation rod 1 to be clamped (fixed) at a position where the interlayer direction (direction of the boundary surface) 2a of the lamination intersects (is offset from) the clamping direction. As a result, a shear force of the component in the interlayer direction (direction of the boundary surface) of the lamination is reduced, and thus it is possible to suppress failure due to the shear.

As illustrated in FIGS. 4A to 5B, in the fixation rod 1 according to the embodiment of the present invention, the marking portion 7 is formed as either an indication portion (examples in FIGS. 4A, 4B, 4C, and 4D) by which the clamping location can be specified or a shape portion (the examples shown in FIGS. 6A and 6B described below) by which the clamping location can be specified. Here, in the fixation rod 1 according to the embodiment of the present invention, the indication portion is a marker provided on an outer peripheral surface of the fixation rod. Examples of the marker include a form as illustrated in FIG. 4A described above, a plurality of lines in the axial direction as illustrated in FIG. 4B (lines in the axial direction of the fixation rod 1), a plurality of oblique lines as illustrated in FIG. 4C (lines inclined with respect to the lines in the axial direction of the fixation rod 1), and a plurality of first oblique lines (lines inclined at a first angle with respect to the lines in the axial direction of the fixation rod 1) and a plurality of second oblique lines (lines inclined at a second angle with respect to the lines in the axial direction of the fixation rod 1) as illustrated in FIG. 4D. Various forms such as solid lines, dotted lines, broken lines, single lines, and multiple lines may be used as the marker of the indication portion, and a seal or a colored coating (screen printing) may be used in addition to a laser marker. The marker of the indication portion may be applied directly to the fixation rod 1 itself (outer surface of the fixation rod 1), applied after a coating is provided on the outer surface of the fixation rod 1, or applied to the fixation rod 1 itself (outer surface of the fixation rod 1) in a manner such that the marker is not exposed on the surface and then covered (in the case of "covering", a transparent resin may be used as the coating). As a material for the coating, a thermoplastic PEEK resin, nylon, polypropylene (PP), and the like may be used, but the material is not limited thereto. In this manner, since the clamping (fixing) position of the fastening member (the rod fixing member 20 described above) with respect to the fixation rod 1 can be easily recognized, this facilitates an operation by an operator and also allows the fixation rod 1 to be clamped (fixed) at the position where the interlayer direction (direction of the boundary surface) 2a of the lamination intersects (is offset from) the clamping direction. As a result, a shear force of the component in the interlayer direction (direction of the boundary surface) 2a of the lamination is reduced, and thus it is possible to suppress failure due to the shear.

As illustrated in FIGS. 6A and 6B, in the fixation rod 1 according to the embodiment of the present invention, the marking portion 7 is a shape portion, and the shape portion is a clamping surface (in the case of FIG. 6A) or a curved shape (in the case of FIG. 6B) provided on the outer peripheral surface of the fixation rod 1. In the fixation rod 1 according to the embodiment of the present invention, the curved shape includes a shape curved in an arcuate shape. In this manner, in the case of a flat surface as illustrated in FIG. 6A, the fixation rod 1 can be stably clamped (fixed) by the fastening member (the rod fixing member 20 described above) because the fixing position is a flat surface. In the case of the curved shape as illustrated in FIG. 6B, by clamping (fixing) the fixation rod 1 toward a bending direction, a direction in which a shear force acts becomes offset from the clamping direction (direction of the boundary surface), and thus shear failure is suppressed. Since the fixation rod 1 is bent toward a laminated direction, a direction of the shear force acting as the fixation rod 1 swings with the clamping location by the fastening member as a fulcrum becomes offset from the laminated direction, and thus it is possible to suppress the occurrence of failure due to the shear force. In particular, by continuously bending the fixation rod in an arcuate shape and shifting the clamping direction, it is possible to suppress the occurrence of failure caused by a localized increase in shear force. Here, although the example illustrated in FIG. 6B is described based on a case in which a part of the fixation rod 1 is bent, the entire fixation rod 1 may alternatively be formed to be bent. According to the fixation rod 1 according to the embodiment of the present invention, since a clamping (fixing) position of the fastening member (the rod fixing member 20 described above) with respect to the fixation rod 1 can be easily recognized, this facilitates an operation by an operator and also allows the fixation rod 1 to be clamped (fixed) at a position where the interlayer direction (direction of the boundary surface) of the lamination intersects (is offset from) the clamping direction. As a result, a shear force of the component in the interlayer direction (direction of the boundary surface) of the lamination is reduced, and thus it is possible to suppress failure due to the shear.

Dimensions, materials, and arrangements of the components described in this specification are not limited to those explicitly described in the embodiments, and the components may be modified to have any dimensions, materials, and arrangements that may fall within the scope of the present invention. In addition, components not explicitly described in the present specification can also be added to the described embodiments, or some of the components described in the embodiments can be omitted.

### Reference Signs List

1 fixation rod
2 reinforcing fiber layer (fiber layer)
2a interlayer direction (direction of boundary surface)
3 resin layer
4 reinforcing fiber layer made of fibers different from fibers of reinforcing fiber layer
5 reinforcing fiber layer having fibers obliquely oriented with respect to fiber direction of reinforcing fiber layer
6 coating layer
7 marking portion
10 spinal fixation device
18 screw member
20 rod fixing member
21 recess portion
22 pressing member

## Claims

1. A fixation rod clamped by a fastening member fastened to a spine,
wherein a plurality of fiber layers are formed such that the plurality of fiber layers are laminated to overlap in a radial direction of the fixation rod as viewed in a cross section perpendicular to an axial direction of the fixation rod,
a marking portion indicating a clamping location by the fastening member is formed in the fixation rod, and
the marking portion is configured to indicate a clamping location in which a clamping direction by the fastening member at the clamping location is a direction intersecting an interlayer direction of the plurality of fiber layers.

2. The fixation rod according to claim 1,
wherein the marking portion is formed as an indication portion capable of specifying the clamping location or a shape portion capable of specifying the clamping location.

3. The fixation rod according to claim 2,
wherein the indication portion is a marker provided on an outer peripheral surface of the fixation rod.

4. The fixation rod according to claim 2,
wherein the shape portion is a clamping surface or a curved shape provided on an outer peripheral surface of the fixation rod.

5. The fixation rod according to claim 4,
wherein the curved shape includes a shape curved in an arcuate shape.
